# EUROPEAN PATENT SPECIFICATION

(11) **EP 2 010 828 B1**
(45) Date of publication and mention of the grant of the patent: **12.11.2014**
(21) Application number: 07733998.4
(22) Date of filing: 16.03.2007
(51) Int. Cl.: F24F 3/16, F24F 1/00, A61L 9/015

(54) **APPLIANCE FOR THE PURIFICATION OF AIR AND MICROBIOLOGICAL SAFETY IN CLOSED ENVIRONMENTS**
GERÄT ZUR LUFTREINIGUNG UND MIKROBIOLOGISCHEN SICHERHEIT IN GESCHLOSSENEN UMGEBUNGEN
APPAREILS DE CONDITIONNEMENT, REGULATION DE L'HUMIDITE RELATIVE, PURIFICATION DE L'AIR ET INNOCUITE MICROBIOLOGIQUE DANS DES ENVIRONNEMENTS FERMES, EN PARTICULIER POUR DES BUREAUX, DES ENVIRONNEMENTS PUBLICS, DES ENVIRONNEMENTS INDUSTRIELS OU AUTRES DU MEME GENRE

(30) Priority: 16.03.2006 IT MO20060084
(43) Date of publication of application: 07.01.2009
(73) Proprietor: Evergreen Tecno Plants S.R.L., 16121 Genova (GE) (IT)
(72) Inventor: BUCCHERI, Antonio, I_36077 Altavilla Vicentina (VI) (IT)
(74) Representative: Brunacci, Marco
(86) International application number: PCT/IB2007/000658
(87) International publication number: WO 2007/105099

(56) References cited:
- JP-A- 5 322 220
- JP-A- 11 076 762
- US-A- 5 788 930
- US-A1- 2004 262 241

## Description

### Technical Field

The present invention refers to an appliance for the conditioning, the control of relative humidity, the purification of air and microbiological safety in closed environments, particularly for offices, dwelling houses, public premises, industrial environments, surgeries, kitchens, ships, public vehicles, small workshops or the like.

### Background Art

It is known that in the aforementioned environments, appliances are commonly used for treating air that permit purifying the air of polluting substances and/or substances that are harmful for people remaining inside such environments both by means of mechanical filtration and by means of ionisation.

Some types of appliances recycle the air and cause it to flow through a series of filters, of the mechanical and/or electrostatic type, on which the particles in suspension remain trapped.

Although in some cases, these appliances are distinguished by a particularly high retention efficiency of the polluting substances, they do however have a number of drawbacks, including the need for frequent maintenance jobs to clean and replace the filters with operating costs that are generally speaking not negligible.

In this respect, it should be remembered that failure to change the filters at the right time results in the ceasing of their filtering effect and can cause the proliferation and spread of particles in suspension in the air, including spores and bacteria.

Nor should it be forgotten either that each traditional filter, quite apart from the degree of filtration it allows achieving, has the sole purpose of trapping the impurities in the air and not making them harmless from a bacteriological viewpoint.

Alternatively to the above-mentioned systems, purifying appliances can be used that avail themselves of the known disinfesting properties of ozone to eliminate pathogenic micro-organisms such as viruses, bacteria, mildew, yeasts and parasites present in the air; ozone (molecule O₃), in fact, is one of the natural disinfesting substances thanks to the high oxidisation potential that distinguishes it.

In point of fact, ozone destroys and deactivates the pathogenic micro-organisms with which it comes into contact; on the one hand by disturbing their growth and on the other by preventing their vital functions.

The ozone first of all damages the cellular wall of the micro-organisms and, then, once it has penetrated inside them, it causes the oxidisation of the essential components such as, enzymes, proteins, etc.

The artificial ozone production technology, initially developed to purify water and now also used to purify the air, can be of two different types; one provides the use of extremely high power voltages (the so-called "corona discharge" systems) and the other the use of ultra-violet rays.

In both cases, the oxygen molecules (O₂) in the air that undergoes the treatment are decomposed which results in the formation of oxygen radicals (O⁻) that can bind with other oxygen molecules to form ozone (O₃).

Particular types of machinery that make use of ozone to sanitize the air, provide special ozone dispensing systems that diffuse such substance inside the environments (offices, homes, etc.) to be purified.

To achieve successful disinfecting action, however, very high quantities of ozone must be diffused in the air, and in some cases this can harm people if inhaled for prolonged periods of time.

In conditions of normal operation, therefore, such machinery of known type provides the dispensing of ozone once the people have left the relevant environments to be purified, and have particular air ventilation systems which, at the end of the treatment, permit expelling the ozone-rich air from the purified environments and introducing other air without ozone taken from the outside. Such machinery too is not without its drawbacks, among which the fact must be recalled that it is rather complex in terms of function and construction because it requires a complicated and cumbersome air ventilation system, which is quite costly.

In this respect, the fact is underlined that the ventilation system, besides having the equipment for air transfer (fans, aspirators or the like), inconveniently requires specific instruments (filters, diaphragms or the like), that allow purifying the air entering the environments and prevent the inopportune introduction of non-purified air thwarting the previous disinfestation done by means of the production of ozone.

Furthermore, producing ozone and expelling this into the atmosphere is not allowed by the current applicable regulations.

JP 11 076 762 discloses an appliance for the purification of air and microbiological safety according to the preamble of Claim 1.

### Object of the Invention

The main aim of the present invention is to provide an appliance for the conditioning, the control of relative humidity, the purification of air and microbiological safety in closed environments, particularly for offices, public environments, industrial environments or the like, that not only allows treating the air and trapping the impurities in it, but also deactivating bacteria, viruses, spores or similar micro-organisms also present on the surfaces inside the environments.

A further object of the present invention is to obtain the above functions in a particularly simple way in terms of structure and management.

Not the last object of the present invention is to provide very effective and efficient use.

Another object of the present invention is that of being particularly attractive from an economic viewpoint, its manufacture being possible with particularly low production costs as well as not requiring high expenditure in terms of installation, operation (low energy consumption), maintenance, environment friendliness and compliance with regulations.

The aforementioned objects are all achieved by the present appliance according to claim 1.

### Brief description of the drawings

Further characteristics and advantages of the present invention will appear even more evident from the detailed description of a preferred, but not exclusive, embodiment of an appliance for the conditioning, the control of relative humidity, the purification of air and microbiological safety in closed environments, particularly for offices, public environments, industrial environments or the like, illustrated indicatively by way of non limiting example, in the attached drawings wherein:

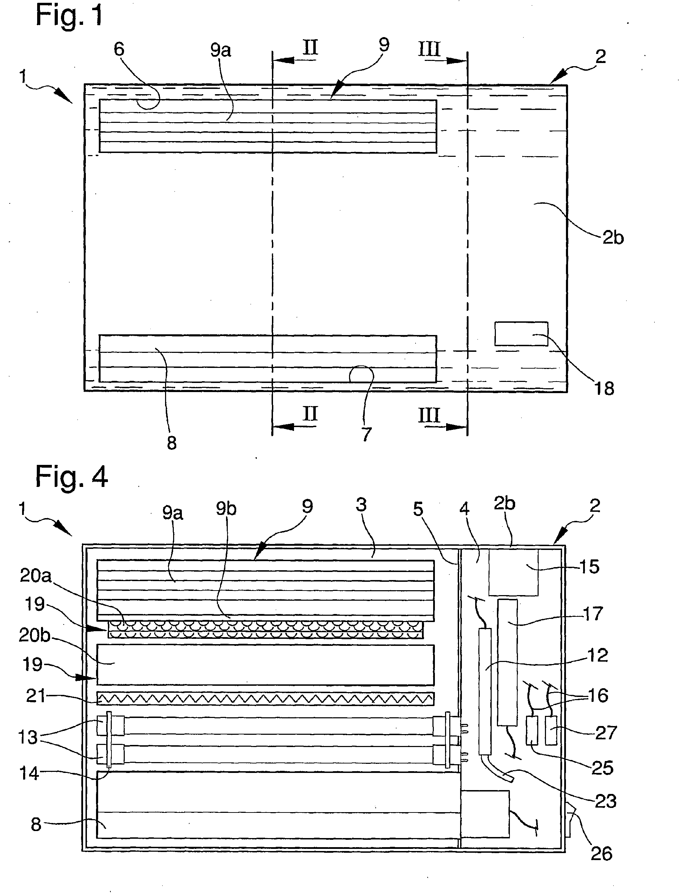

the figure 1 is a front, schematic and partial view of the appliance according to the invention;

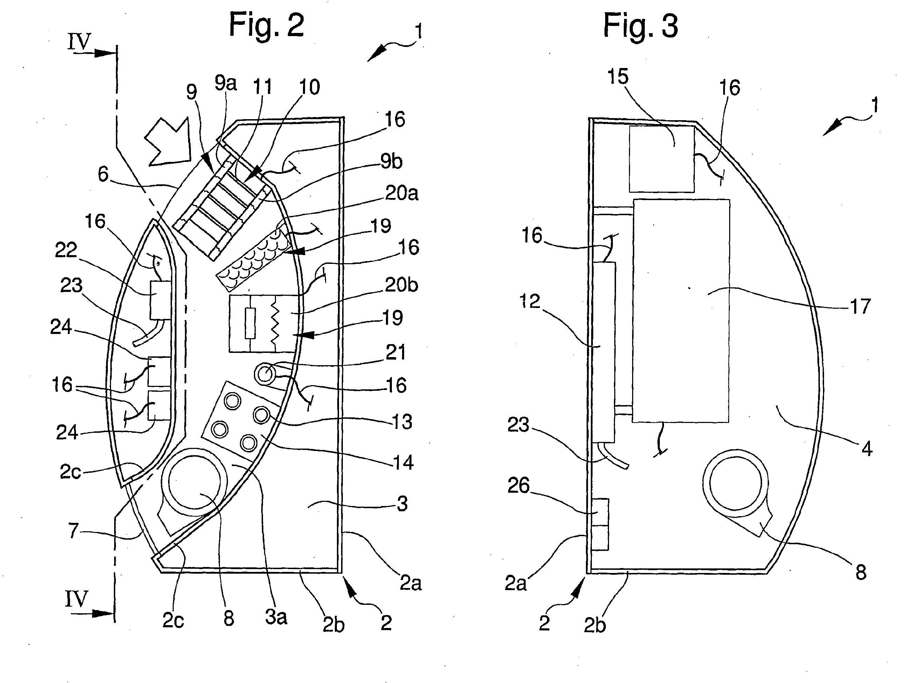

the figure 2 is a section, schematic and partial view, obtained on the trace plane II - II of figure 1;
the figure 3 is a section, schematic and partial view, obtained on the trace plane III - III of figure 1;
the figure 4 is a section, schematic and partial view, obtained on the trace plane IV - IV of figure 2.

### Embodiments of the Invention

With special reference to such figures, an appliance has been globally designated by reference number 1 for the conditioning, the control of relative humidity, the purification of air and microbiological safety in closed environments, particularly for offices, public environments, industrial environments or the like.

The appliance 1 comprises a base structure 2, hollow inside and made up of a vertical plate 2a, that allows it to be wall-mounted in the environment to be treated, and a closing case 2b, of box shape, associable with the vertical plate 2a.

When assembled, the structure 2 defines within it a first compartment 3 and a second compartment 4, separated from one another by a cross septum 5.

The structure 2 has an inlet opening 6 for the air to be treated, which is obtained at the substantially upper portion of the case 2b, and an outlet opening 7 for the treated air, which is obtained at the substantially lower portion of the case 2b. Each opening 6 and 7 has a respective protection grille, not shown in the figures.

The inlet and outlet openings 6 and 7 both face onto the first compartment 3 of the structure 2 and are placed in communication the one with the other by means of a pair of arched sheets 2c which define a channel 3a for the flow of the air, which extends through the first compartment 3 from the inlet opening 6 to the outlet opening 7.

Inside the channel 3a are fitted movement means 8 for moving the air, suitable for extracting the air through the inlet opening 6 and pushing it towards the outlet opening 7.

The movement means 8 consist, for example, of a tangential fan fitted on the surface of one of the arched sheets 2c near the outlet opening 7.

At the inlet opening 6 are associated mechanical air filtration means 9, comprising a first mesh filter 9a and a second mesh filter 9b, suitable for trapping the solid particles and the coarser impurities in suspension in the air. Between the first and the second mesh filter 9a and 9b are other air filtration means, this time of the electrostatic type, altogether indicated by the reference number 10.

The electrostatic filtration means 10 are composed of a series of laminae 11 that extend from the inlet opening 6 towards the inside of the first compartment 3 and which, once energised, generate an electric field crossed by the inflowing air.

Such electric field first of all causes the ionisation of the air and of the finer solid particles in suspension, which are then captured and collected by electrostatic attraction on the surfaces of the laminae 11.

Downstream of the electrostatic filtration means 10 with respect to the direction of movement of the air through the channel 3a, are positioned heat exchange means 19 suitable for cooling or heating the inflowing air.

The heat exchange means 19 comprise a thermal battery 20a which is arranged inside the channel 3a and is connected to a compression and condensation unit, arranged outside the appliance 1 and not shown in the figures.

The thermal battery 20a is able to cool the environment in summer and, by means of a heat pump, to heat in winter or whenever necessary.

Furthermore, the heat exchange means 19 have an absorption refrigeration circuit 20b fitted directly on board the appliance 1, along the channel 3a and close to the thermal battery 20a.

In the particular embodiment of the invention shown in the figures, the thermal battery 20a and the absorption refrigeration circuit 20b are suitable for cooperating together to thermally condition the air flowing through the channel 3a.

Different embodiments of this invention cannot be ruled out in which only one of either the thermal battery 20a or the absorption refrigeration circuit 20b is present, or in which the appliance 1 is connected to a number of air distribution ducts that permit making a version of the appliance 1 with "free-cooling" operation, i.e., able to exploit the low atmospheric temperatures, for example in winter, for the purpose of cooling the environment to be treated.

Furthermore, downstream of the heat exchange means 19, de-humidifying means 21 are arranged, of the electric resistor type able to dehumidify the air flowing in the appliance 1 (post-heating function).

On one of the arched sheets 2c, on the opposite side with respect to the channel 3a, a compressor 22 is fitted, suitable for drying the air and allowing air movement between the channel 3a and ozonizing means 12 suitable, in a first purification phase, for transforming the molecular oxygen (O₂) of the air coming from the environment into ozone (O₃).

The ozonizing means 12 consist, for example, of a corona discharge ozone generator, which is positioned in the compartment 4 and which is in fluidic communication with the compressor 22 and the channel 3a by means of a series of communication ducts 23.

Different embodiments of the present invention cannot however be ruled out in which the ozonizing means 12 are composed of ultra-violet ray emitting ozonizing lamps, housed along the channel 3a.

Close to the outlet opening 7, catalyzer means 13 are arranged, suitable for realising a reverse reaction with respect to the ozonization and which consists in transforming into molecular oxygen (O₂) the ozone (O₃) contained in the air coming from the environment.

In the particular embodiment of the invention shown in the figures, the catalyzer means 13 are composed of ultra-violet ray emitting catalyzation lamps, fitted inside the channel 3a on brackets 14 associated with the arched sheets 2c.

In particular, the catalyzation lamps 13 are UV lamps of the so-called "germicide" type, i.e., able to emit ultra-violet rays at particular wavelengths which, besides performing a catalytic reduction action of the ozone (O₃) into molecular oxygen (O₂), allow lowering the bacterial load in the irradiated air. During use, the catalyzation lamps 13 are activated by means of a series of reactors 24 supported by the arched sheets 2c underneath the compressor 22. Alternatively or together with the catalyzation lamps 13, the system usable for transforming into molecular oxygen (O₂) the ozone (O₃) contained in the air coming from the environment could comprise a catalyzation system of the thermal type.

The appliance 1 also comprises power supply means 15 for supplying the fan 8, the laminae 11, the thermal battery 20a, the absorption refrigeration circuit 20b, the electric resistor 21, the corona discharge ozone generator 12 and the catalyzation lamps 13.

Such power supply means, of the type of a supply unit that can be connected to the normal power mains, are fitted inside the second compartment 4, associated with the structure 2, and connected to the different units by means of a power connection circuit 16, not shown in detail in the figures.

Inside the second compartment 4 an inverter 25 is also fitted, that ensures the energy-saving operation of the appliance 1.

To manage the operation of the appliance 1 a processing and control unit 17 is provided, of the electronic board type or the like, which is fitted underneath the supply unit 15 and which, by means of the power connection circuit 16 is operatively associated with the supply unit 15, with the electrostatic filtration means 10, with the thermal battery 20a, with the absorption refrigeration circuit 20b, with the electric resistor 21, with the corona discharge ozone generator 12, with the catalyzation lamps 13, and with the inverter 25.

On one of the side walls of the case 2b an on/off switch 26 is positioned, having a protection fuse and controllable from outside to switch power to the appliance 1 on/off.

Moreover, inside the compartment 4 power supply cut-off means are fitted, composed for example of a microswitch 27 suitable for cutting off the power supply to the appliance 1 at the same time as the removal/opening of the case 2b.

Usefully, the activity of the processing and control unit 17 is interlocked with sensor means suitable for detecting the concentration of ozone in the environment, and/or electronic means for detecting the bacterial loads in the air or on the surfaces. Depending on the quantity of ozone and bacteria in the air or on the surfaces, in point of fact, the operation of the appliance 1 can change in real time and consequently increase or reduce the production of ozone.

The processing and control unit 17 interacts operatively on the one hand with a remote programming and control device, e.g., of the type of a remote-control, to switch on/switch off/set the appliance 1, and on the other hand with transmission and remotization means of the alarm signals and/or the operating parameters of the processing and control unit 17, e.g., of the type of an IT system with remote interface, a satellite transmitter, a telephone exchange, or the like.

The sensor means, the electronic detection means, the programming and control device and the transmission and remotization means are not shown in detail in the figures.

The appliance 1 is completed by specific displaying means 18 for displaying the operating parameters of the processing and control unit 17; these displaying means consist, for example, of a display screen mounted on the case 2b and which, by means of the power connection circuit 16, is operatively connected to the electronic board 17.

In combination or alternatively to the display screen 18, a device can be fitted for the remote display of the controls of the processing and control unit 17, of the type of a display mounted on the remote-control or the like.

The operation of the present invention is the following.

During the first phase of the operating cycle of the appliance 1, which is mounted stably on a wall of the environment where the air is to be treated, and is electrically connected to the normal power mains, the fan 8 is operating together with the electrostatic filtration means 10 and with the corona discharge ozone generator 12, while the catalyzation lamps 13, the thermal battery 20a and the absorption refrigeration circuit 20b are not operative.

In this phase, the room air is moved by the fan 8 and crosses the channel 3a in the first compartment 3, enriching itself with ozone (O₃) produced by the corona discharge ozone generator 12, which by means of an electrode splits the molecular oxygen (O₂) of the air into two atoms of oxygen (O), which combine in turn with other molecular oxygen (O₂) thereby producing ozone (O₃).

The outlet opening 7 is therefore crossed by a more or less continuous flow of ozone which spreads through the environment, increasing its relevant concentration until it reaches limit values beyond which the death is caused of the pathogenic micro-organisms.

At the end of this first phase, the duration of which can be set by means of the electronic board 17, the second catalyzation phase starts during which the only energised and therefore switched-on parts of the appliance 1 are the fan 8, the electrostatic filtration means 10 and the catalyzation lamps 13.

In this second phase the air in the environment is re-circulated inside the structure 2 and the ozone (O₃) still remaining in it is reconverted into molecular oxygen (O₂) by means of a reverse process to the previous one.

In this way, at the end of the disinfecting cycle the concentration of ozone in the environment is practically nil.

It must be observed that during the above cycle, no one must be present inside the environment (inside an office or other work premises in which normal workday activities are performed during the day, for example, such cycle can be advantageously run during the night hours).

Besides the ozone disinfecting cycle, during the remaining hours of the day when the environment is lived in by people, the appliance 1 is still able to perform the germicide treatment with the ultraviolet catalyzation lamps 13; the mechanical and electrostatic filtration of the air by means of the specific filtration means 9 and 10, in which are collected the impurities present in the air, which also include the viruses, the bacteria, the mildews and the other micro-organisms harmful for the health of human beings which can be definitively eliminated once the ozone disinfecting cycle has been started again; the conditioning of the environment by means of the thermal battery 20a and the absorption refrigeration circuit 20b; the control of relative humidity by means of the operation of the electric resistor 21.

In all functions the appliance 1 is particularly effective as regards the bacteriological control of the environments achieved by means of the synergy between disinfestation through ozone and the mechanical and electrostatic filtration of the air.

It has in fact been found that the described invention achieves the proposed objects.

## Claims

1. Appliance (1) for the purification of air and microbiological safety in closed environments, particularly for offices, public environments, industrial environments or the like, comprising :
- an internally hollow base structure (2) having at least one inlet opening (6) for the air to be treated coming from a substantially closed environment, and at least one outlet opening (7) for the treated air,
inside said structure (2) there being provided :
- movement means for moving the air from said inlet opening (6) to said outlet opening (7),
- ozonizing means (12) for ozonizing the air, suitable, in a first purification phase, for transforming the molecular oxygen of the air coming from said environment into ozone, and
- catalyzer means (13) suitable, in a second purification phase following said first phase, for transforming the ozone of the air coming from said closed environment into molecular oxygen,
wherein said appliance also comprises ozone sensor means suitable for detecting the ozone concentration in the closed environment; and
- a processing and control unit (17) operatively associated with said movement means (8), said ozonizing means (12), said catalyzer means (13), and said ozone sensor means,
said appliance (1) being **characterized in that** it further comprises bacteria sensor means suitable for detecting the bacterial load in the air and/or on the adjacent surfaces,
wherein said processing and control unit (17) is provided for controlling in real time the production of ozone by said ozonizing means (12), depending on the quantity of ozone and of the bacteria load in the air and/or on the surfaces, as detected respectively by said ozone sensor means and by said bacteria sensor means, and
wherein said processing and control unit (17) is provided for setting the duration of said first purification phase and, at the end of the same, for starting said second purification phase by switching-off said ozonizing means (12) and by switching-on said catalyzer means (13), and for energizing said movement means (8) during said second purification phase.

2. Appliance (1) according to claim 1, **characterized in that** said structure (2) comprises at least one wall-mounted plate (2a) and a box-shaped closing case (2b) associable with said plate (2a), whereby said appliance (1) is suitable for being wall-mounted in the environment to be treated.

3. Appliance (1) according to claims 1 or 2, **characterized in that** said structure (2) defines at its inside at least one channel (3a) for the flow of the air, which extends from said inlet opening (6) to said outlet opening (7),
wherein both said ozonizing means (12) and said catalyzer means (13) are associated with said one channel (3a) for treating the air flowing along it and coming from said environment through said at least one inlet opening (6).

4. Appliance according to one or more of the preceding claims, **characterized in that** it further comprises air filtration means (9), in particular of the electrostatic type or mechanical type, arranged inside said structure (2) close to said inlet opening (6).

5. Appliance according to one or more of the preceding claims, **characterized in that** it comprises heat exchange means (19) suitable for cooling and/or heating the air,
wherein in particular said heat exchange means (19) comprise at least one of :
- a thermal battery (20a) fitted inside said structure (2) and suitable for being connected to a compression and condensation unit, arranged outside said structure (2);
- one absorption refrigeration circuit (20b) fitted inside said structure (2); and
- an air distribution duct suitable for "free-cooling" operation.

6. Appliance according to one or more of the preceding claims, **characterized in that** it comprises air de-humidifying means (21),
wherein in particular said de-humidifying means (21) comprise at least one electric resistor arranged inside said structure (2).

7. Appliance according to one or more of the preceding claims, **characterized in that** said ozonizing means (12) comprise at least one ozone generator of the corona discharge type.

8. Appliance according to one or more of the preceding claims, **characterized in that** said catalyzer means (13) are arranged inside said structure (2) close to said outlet opening (7).

9. Appliance according to one or more of the preceding claims, **characterized in that** said catalyzer means (13) comprise at least one of:
- an ultra-violet ray emitting catalyzation lamp;
- a UV lamp of the "germicide" type; and
- a thermal catalyzation system.

10. Appliance according to one or more of the preceding claims, **characterized in that** said movement means (8) comprise at least one fan, in particular of the tangential type.

11. Appliance according to one or more of the preceding claims, **characterized in that** it comprises power supply means (15, 16) of at least one of said movement means, said ozonizing means (12) and said catalyzer means (13).

12. Appliance according to claims 4 or 5 or 6 or 11, **characterized in that** it said processing and control unit (17) is operatively associated with respectively said air filtration means (9) of the electrostatic type, or said heat exchange means (19), or said de- humidifying means (21), or said power supply means (15).

13. Appliance according to one or more of the preceding claims, **characterized in that** it comprises at least one of:
- transmission and remotization means of the alarm signals and/or the operating parameters of said processing and control unit (17); and
- a remote programming and control device of said processing and control unit (17).

14. Appliance according to one or more of the preceding claims, **characterized in that** it comprises displaying means, associated with said structure (2), for displaying the operating parameters of said processing and control unit (17)

15. Appliance according to one or more of the preceding claims, **characterized in that** at the side walls of said structure (2) there is provided at least one on/off switch, having a protection fuse and controllable from outside to switch power on/off.

## Patentansprüche

1. Gerät (1) für die Reinigung von Luft und mikrobiologische Sicherheit in geschlossenen Umgebungen, insbesondere für Büros, öffentliche Umgebungen, industrielle Umgebungen oder dergleichen, umfassend:
- eine interne hohle Grundstruktur (2), die mindestens eine Einlassöffnung (6) für die zu behandelnde Luft, die von einer im Wesentlichen geschlossenen Umgebung kommt, und mindestens eine Auslassöffnung (7) für die behandelte Luft aufweist, wobei innerhalb der Struktur (2) vorgesehen sind:
- Bewegungsmittel zum Bewegen der Luft von der Einlassöffnung (6) zu der Auslassöffnung (7),
- Ozonisierungsmittel (12) zum Ozonisieren der Luft, die zum Umwandeln des molekularen Sauerstoffs der Luft, die von der Umgebung kommt, in Ozon in einer ersten Reinigungsphase geeignet sind, und
- Katalysatormittel (13), die zum Umwandeln des Ozons der Luft, die von der geschlossenen Umgebung kommt, in molekularen Sauerstoff in einer der ersten Reinigungsphase folgenden zweiten Reinigungsphase geeignet sind,
wobei das Gerät außerdem Ozonsensormittel umfasst, die zum Detektieren der Ozonkonzentration in der geschlossenen Umgebung geeignet sind; und
- eine Steuerungs- und Regelungseinheit (17), die betriebsfähig mit den Bewegungsmitteln (8), den Ozonisierungsmitteln (12), den Katalysatormitteln (13) und den Ozonsensormitteln verbunden sind,
wobei das Gerät (1) **dadurch gekennzeichnet ist, dass** es ferner Bakteriensensormittel umfasst, die zum Detektieren der Bakterienbelastung in der Luft und/oder auf angrenzenden Oberflächen geeignet sind,
wobei die Steuerungs- und Regelungseinheit (17) dazu eingerichtet ist, die Production von Ozon durch die Ozonisierungsmittel (12) in Echtzeit und abhängig von der Quantität von Ozon und der Bakterienbelastung in der Luft und/oder auf den Oberflächen zu regeln, wie sie jeweils von den Ozonsensormitteln und von den Bakteriensensormitteln detektiert wurden, und
wobei die Steuerungs- und Regelungseinheit (17) zum Einstellen der Dauer der ersten Reinigungsphase und am Ende derselben zum Starten der zweiten Reinigungsphase durch Ausschalten der Ozonisierungsmittel (12) und durch Einschalten der Katalysatormittel (13) und zum Antreiben der Bewegungsmittel (8) während der zweiten Reinigungsphase eingerichtet ist.

2. Gerät (1) nach Anspruch 1, **dadurch gekennzeichnet, dass** die Struktur (2) mindestens eine Wandplatte (2a) und ein kastenförmiges Abschlussgehäuse (2b), das mit der Platte (2a) verbindbar ist, umfasst, wobei das Gerät (1) geeignet ist, um an einer Wand in der zu behandelnden Umgebung montiert zu werden.

3. Gerät (1) nach den Ansprüchen 1 oder 2, **dadurch gekennzeichnet, dass** die Struktur (2) in ihrem Inneren mindestens einen Kanal (3a) für den Strom der Luft definiert, der sich von der Einlassöffnung (6) zu der Auslassöffnung (7) erstreckt,
wobei sowohl die Ozonisierungsmittel (12) als auch die Katalysatormittel (13) mit dem einen Kanal (3a) zum Behandeln der Luft verbunden sind, die daran entlang strömt und von der Umgebung durch die mindestens eine Einlassöffnung (6) kommt.

4. Gerät nach einem oder mehreren der vorherigen Ansprüche, **dadurch gekennzeichnet, dass** es ferner Luftfilterungsmittel (9), insbesondere des elektrostatischen Typs oder des mechanischen Typs, umfasst, die innerhalb der Struktur (2) nahe der Einlassöffnung (6) angeordnet sind.

5. Gerät nach einem oder mehreren der vorherigen Ansprüche, **dadurch gekennzeichnet, dass** es Wärmeaustauschmittel (19) umfasst, die zum Kühlen und/oder Erwärmen der Luft geeignet sind,
wobei insbesondere die Wärmeaustauschmittel (19) mindestens eines umfassen von:
- eine Wärmebatterie (20a), die innen in die Struktur (2) eingepasst und geeignet ist, um mit einer Kompressions- und Kondensationseinheit verbunden zu werden, die außerhalb der Struktur (2) angeordnet ist;
- ein Absorptionskühlkreis (20b), der innen in die Struktur (2) eingepasst ist; und
- ein Luftverteilerkanal, der für einen "free-cooling"-Betrieb geeignet ist.

6. Gerät nach einem oder mehreren der vorherigen Ansprüche, **dadurch gekennzeichnet, dass** es Luftentfeuchtungsmittel (21) umfasst,
wobei insbesondere die Luftentfeuchtungsmittel (21) mindestens einen elektrischen Widerstand umfassen, der innen in der Struktur (2) angeordnet ist.

7. Gerät nach einem oder mehreren der vorherigen Ansprüche, **dadurch gekennzeichnet, dass** die Ozonisierungsmittel (12) mindestens einen Ozongenerator des Koronaentladungstyps umfasst.

8. Gerät nach einem oder mehreren der vorherigen Ansprüche, **dadurch gekennzeichnet, dass** die Katalysatormittel (13) innen in der Struktur (2) nahe der Auslassöffnung (7) angeordnet sind.

9. Gerät nach einem oder mehreren der vorherigen Ansprüche, **dadurch gekennzeichnet, dass** die Katalysatormittel (13) mindestens eines umfassen von:
- eine ultra-violette Strahlen emittierende Katalysatorlampe;
- eine UV-Lampe des "Germizid"-Typs; und
- ein thermisches Katalysatorsystem.

10. Gerät nach einem oder mehreren der vorherigen Ansprüche, **dadurch gekennzeichnet, dass** die Bewegungsmittel (8) mindestens einen Lüfter, insbesondere des tangentialen Typs, umfassen.

11. Gerät nach einem oder mehreren der vorherigen Ansprüche, **dadurch gekennzeichnet, dass** es Energieversorgungsmittel (15, 16) von mindestens einem der Bewegungsmittel, der Ozonisierungsmittel (12) und der Katalysatormittel (13) umfasst.

12. Gerät nach den Ansprüchen 4 oder 5 oder 6 oder 11, **dadurch gekennzeichnet, dass** die Steuerungs- und Regelungseinheit (17) jeweils betriebsfähig mit den Luftfilterungsmitteln (9) des elektrostatischen Typs oder den Wärmeaustauschmitteln (19) oder den Entfeuchtungsmitteln (21) oder den Energieversorgungsmitteln (15) verbunden sind.

13. Gerät nach einem oder mehreren der vorherigen Ansprüche, **dadurch gekennzeichnet, dass** es mindestens eines umfasst von:
- Übertragungs- und Fernbedienungsmittel der Alarmsignale und/oder der Betriebsparameter der Steuerungs- und Regelungseinheit (17); und
- ein Fernprogrammier- und Steuergerät der Steuerungs- und Regelungseinheit (17).

14. Gerät nach einem oder mehreren der vorherigen Ansprüche, **dadurch gekennzeichnet, dass** es Anzelgemittel zum Anzeigen der Betriebsparameter der Steuerungs- und Regelungseinheit (17) umfasst, die mit der Struktur (2) verbunden sind.

15. Gerät nach einem oder mehreren der vorherigen Ansprüche, **dadurch gekennzeichnet, dass** an den Seitenwänden der Struktur (2) mindestens ein An-/Aus-Schalter vorgesehen ist, der eine Absicherung aufweist und von außen steuerbar ist, um Energie ein-/auszuschalten.

## Revendications

1. Appareil (1) pour la purification de l'air et l'innocuité microbiologique dans des environnements fermés, en particulier pour des bureaux, des environnements publics, des environnements industriels ou autres du même type, comprenant :
- une structure de base (2) creuse intérieurement, comportant au moins une ouverture d'admission (6) pour l'air qui doit être traité et qui provient d'un environnement pratiquement fermé, et au moins une ouverture de sortie (7) pour l'air traité,
ladite structure (2) étant intérieurement prévue avec :
- des moyens de déplacement pour déplacer l'air de ladite ouverture d'admission (6) jusqu'à ladite ouverture de sortie (7),
- des moyens d'ozonation (12) pour l'ozonation de l'air, aptes, dans une première phase de purification, à transformer en ozone l'oxygène moléculaire de l'air provenant dudit environnement, et
- des moyens de catalyse (13), aptes, dans une deuxième phase de purification qui suit ladite première phase, à transformer en oxygène moléculaire l'ozone de l'air provenant dudit environnement fermé,
dans lequel ledit appareil comprend aussi des moyens de détection d'ozone aptes à détecter la concentration d'ozone dans l'environnement fermé ; et
- une unité de traitement et de commande (17), associée de manière fonctionnelle auxdits moyens de déplacement (8), auxdits moyens d'ozonation (12), auxdits moyens de catalyse (13), et auxdits moyens de détection d'ozone,
ledit appareil (1) étant **caractérisé en ce qu'**il comprend en outre des moyens de détection de bactéries, aptes à détecter la charge bactérienne dans l'air et/ou sur les surfaces adjacentes,
dans lequel ladite unité de traitement et de commande (17) est prévue pour commander en temps réel la production d'ozone par lesdits moyens d'ozonation (12), en fonction de la quantité d'ozone et de la charge bactérienne dans l'air et/ou sur les surfaces, telles que détectées respectivement par lesdits moyens de détection d'ozone et par lesdits moyens de détection de bactéries, et
dans lequel ladite unité de traitement et de commande (17) est prévue pour régler la durée de ladite première phase de purification et, à la fin de cette dernière, pour démarrer ladite deuxième phase de purification par coupure desdits moyens d'ozonation (12) et par enclenchement desdits moyens de catalyse (13), puis pour activer lesdits moyens de déplacement (8) pendant ladite deuxième phase de purification.

2. Appareil (1) selon la revendication 1, **caractérisé en ce que** ladite structure (2) comprend au moins une plaque (2a) montée sur une paroi et un étui de fermeture (2b) en forme de boîte pouvant être associé à ladite plaque (2a), ledit appareil (1) étant, de ce fait, apte à être monté sur une paroi dans l'environnement qui doit être traité.

3. Appareil (1) selon les revendications 1 ou 2, **caractérisé en ce que** ladite structure (2) définit dans sa partie intérieure au moins un canal (3a) pour l'écoulement de l'air, qui s'étend de ladite ouverture d'admission (6) jusqu'à ladite ouverture de sortie (7),
dans lequel à la fois lesdits moyens d'ozonation (12) et lesdits moyens de catalyse (13) sont associés audit un canal (3a) pour traiter l'air s'écoulant le long de celui-ci et provenant dudit environnement par l'intermédiaire de ladite au moins une ouverture d'admission (6).

4. Appareil selon une ou plusieurs des revendications précédentes, **caractérisé en ce qu'**il comprend en outre des moyens de filtration d'air (9), en particulier de type électrostatique ou de type mécanique, disposés à l'intérieur de ladite structure (2), au voisinage de ladite ouverture d'admission (6).

5. Appareil selon une ou plusieurs des revendications précédentes, **caractérisé en ce qu'**il comprend des moyens d'échange de chaleur (19) aptes à refroidir et/ou échauffer l'air,
dans lequel, en particulier, lesdits moyens d'échange de chaleur (19) comprennent au moins l'un parmi :
- une batterie thermique (20a) ajustée à l'intérieur de ladite structure (2) et apte à être reliée à une unité de compression et de condensation, disposée à l'extérieur de ladite structure (2) ;
- un circuit de réfrigération et d'absorption (20b) ajusté à l'intérieur de ladite structure (2) ;
et
- un conduit de distribution d'air approprié pour une opération de « libre refroidissement ».

6. Appareil selon une ou plusieurs des revendications précédentes, **caractérisé en ce qu'**il comprend des moyens de déshumidification (21) de l'air,
dans lequel, en particulier, lesdits moyens de déshumidification (21) comprennent au moins une résistance électrique disposée à l'intérieur de ladite structure (2).

7. Appareil selon une ou plusieurs des revendications précédentes, **caractérisé en ce que** lesdits moyens d'ozonation (12) comprennent au moins un générateur d'ozone du type à décharge par effet de couronne.

8. Appareil selon une ou plusieurs des revendications précédentes, **caractérisé en ce que** lesdits moyens de catalyse (13) sont disposés à l'intérieur de ladite structure (2), au voisinage de ladite ouverture de sortie (7).

9. Appareil selon une ou plusieurs des revendications précédentes, **caractérisé en ce que** lesdits moyens de catalyse (13) comprennent au moins l'un parmi :
- une lampe de catalyse à émission d'un rayonnement ultraviolet ;
- une lampe UV de type « germicide » ; et
- un système de catalyse thermique.

10. Appareil selon une ou plusieurs des revendications précédentes, **caractérisé en ce que** lesdits moyens de déplacement (8) comprennent au moins un ventilateur, en particulier de type tangentiel.

11. Appareil selon une ou plusieurs des revendications précédentes, **caractérisé en ce qu'**il comprend des moyens d'alimentation (15, 16) d'au moins l'un parmi lesdits moyens de déplacement, lesdits moyens d'ozonation (12) et lesdits moyens de catalyse (13).

12. Appareil selon les revendications 4 ou 5 ou 6 ou 11, **caractérisé en ce que** ladite unité de traitement et de commande (17) est associée de manière fonctionnelle respectivement auxdits moyens de filtration d'air (9) de type électrostatique, ou auxdits moyens d'échange de chaleur (19), ou auxdits moyens de déshumidification (21), ou auxdits moyens d'alimentation (15).

13. Appareil selon une ou plusieurs des revendications précédentes, **caractérisé en ce qu'**il comprend au moins l'un parmi :
- des moyens de transmission et d'envoi à distance des signaux d'alarme et/ou des paramètres de fonctionnement de ladite unité de traitement et de commande (17) ; et
- un dispositif de programmation et de commande à distance de ladite unité de traitement et de commande (17).

14. Appareil selon une ou plusieurs des revendications précédentes, **caractérisé en ce qu'**il comprend des moyens d'affichage, associés à ladite structure (2), pour afficher les paramètres de fonctionnement de ladite unité de traitement et de commande (17).

15. Appareil selon une ou plusieurs des revendications précédentes, **caractérisé en ce qu'**au niveau des parois latérales de ladite structure (2), il est prévu au moins un commutateur de mise en marche/d'arrêt, comportant un fusible de protection et pouvant être commandé de manière externe pour activer/désactiver l'alimentation.
